# EUROPEAN PATENT APPLICATION

(11) **EP 4 585 682 A1**
(43) Date of publication of application: **16.07.2025**
(21) Application number: 23859337.0
(22) Date of filing: 29.08.2023
(51) Int. Cl.: C12N 9/22, C12N 15/113

(54) **MUTATED V-TYPE CRISPR ENZYME AND USE THEREOF**

(30) Priority: 29.08.2022 CN 202211039436
(71) Applicant: Beijing Synsortech Co., Ltd., Beijing 102600 (CN); Zhejiang Synsorbio Technology Co., Ltd., Shaoxing, Zhejiang 312300 (CN); Beijing Synsorbio Technology Co., Ltd., Beijing 102600 (CN); Zhejiang Synsorbio Gene Technology Co., Ltd., Shaoxing, Zhejiang 312300 (CN)
(72) Inventor: PAN, Weiye, Guangzhou, Guangdong 510405 (CN); SUN, Yang, Beijing 100052 (CN); CHEN, Chongjian, Jinhua, Zhejiang 322006 (CN); ZHU, Pengyu, Beijing 100176 (CN); YOU, Shenghao, Cangzhou, Hebei 061804 (CN)
(74) Representative: karo IP
(86) International application number: PCT/CN2023/115471
(87) International publication number: WO 2024/046307

(57) **Abstract**

Provided are a mutated V-type CRISPR enzyme and use thereof. By means of the protein mutation rational mutagenesis technology, the restriction on the recognition and cleavage of DNA substrates by RuvC in Cas12a or Cas12b is removed, so that RNA substrates can be efficiently recognized and cleaved, and the problem of insufficient RNA substrate recognition efficiency in practical applications is solved. The reaction efficiency of Cas12 with the RNA substrates after the activation of trans-cleavage activity is improved, and the reporting efficiency of Cas12 using an RNA probe is improved.

## Description

### TECHNICAL FIELD

The present application belongs to the field of biotechnology, particularly relates to a mutated V-type CRISPR enzyme and use thereof.

### BACKGROUND

The V-type CRISPR-Cas system is also known as the Cas12 family. It differs from other CRISPR-Cas systems in that: it is an RNA-mediated single-effector ribozyme driven by a single RuvC active center. As more and more Cas12s are discovered and identified, this family now has multiple categories, including V-A, V-B, V-C, V-D, V-E, V-F, V-G, V-H, V-I, V-J, and V-K.

Among them, Cas12a and Cas12b are used in multiple nucleic acid detection technologies and clinical detection products combined with amplification technologies due to their excellent reaction performance.

In previous reports, Cas12a or Cas12b both have RNA-mediated cis-DNase activity and trans-DNase activity, as well as the detection applications emerged as a result; although some reports mention that Cas12a or Cas12b has RNA-mediated trans-RNase activity and can cut RNA substrates with an efficiency of 1/10 that of DNA substrates, its preferential selectivity for DNA substrates limits its further application in nucleic acid amplification systems. This limited discrimination of one order of magnitude makes it possible for RNA probes to be used as an alternative reporter substrate for detection.

### SUMMARY

Aiming at the problems existing in the prior art, the present application provides a mutated V-type CRISPR enzyme and use thereof.

Particularly, this application involves the following aspects:
1. An engineered Cas12 protein or a functional derivative thereof, comprising the following mutations:
   removing the restriction on the recognition and cleavage of DNA substrates by RuvC and/or Nuc active centers in a reference Cas12 protein,
   preferably, the amino acid sequence of the reference Cas12 protein is represented by SEQ ID NO: 1.
2. The engineered Cas12 protein or a functional derivative thereof according to item 1, wherein removing the restriction on the recognition and cleavage of DNA substrates by RuvC and/or Nuc active centers in the reference Cas12 protein is achieved by:
   replacing the fingers upstream and downstream of the α-helix of the gatekeeper amino acids in the reference Cas12 protein with zinc finger domains.
3. The engineered Cas12 protein or a functional derivative thereof according to item 1 or 2, wherein removing the restriction on the recognition and cleavage of DNA substrates by RuvC and/or Nuc active centers in the reference Cas12 protein is replacing the domains in part or all of the region in positions 915-945, wherein the amino acid position numbering is as defined in SEQ ID NO: 1.
4. The engineered Cas12 protein or a functional derivative thereof according to item 2, wherein the zinc finger domain has 1-2 zinc binding sites, and the zinc binding site is any one selected from the group consisting of: [CxxxxC], [CxxxxH], [CxxxC], [HxxxH], [CxxC] and [CxxH], wherein x represents any natural amino acid.
5. The engineered Cas12 protein or a functional derivative thereof according to item 4, wherein the zinc finger domain is a zinc finger domain of Cas12g or HIV-1 nucleocapsid protein 7 (Ncp7).
6. The engineered Cas12 protein or a functional derivative thereof according to item 5, wherein positions 919-944 of the reference Cas12 protein are replaced with the amino acid sequence represented by SEQ ID NO: 4, wherein the amino acid position numbering is as defined in SEQ ID NO: 1.
7. The engineered Cas12 protein or a functional derivative thereof according to item 5, wherein positions 919-944 of the reference Cas12 protein are replaced with the amino acid sequence represented by SEQ ID NO:6, wherein the amino acid position numbering is as defined in SEQ ID NO: 1.
8. The engineered Cas12 protein or a functional derivative thereof according to item 1, wherein positions 919-944 of the reference Cas12 protein are replaced with the amino acid sequence represented by SEQ ID NO: 8, wherein the amino acid position numbering is as defined in SEQ ID NO: 1.
9. The engineered Cas12 protein or a functional derivative thereof according to item 1, wherein positions 916-944 of the reference Cas12 protein are replaced with the amino acid sequence represented by SEQ ID NO: 11, wherein the amino acid position numbering is as defined in SEQ ID NO: 1.
10. An engineered Cas12 protein or a functional derivative thereof, comprising one of the following mutations based on a reference Cas12 protein:
   replacing positions 919-944 of the reference Cas12 protein with the amino acid sequence represented by SEQ ID NO: 4;
   replacing positions 919-944 of the reference Cas12 protein with the amino acid sequence represented by SEQ ID NO: 6;
   replacing positions 919-944 of the reference Cas12 protein with the amino acid sequence represented by SEQ ID NO: 8; and
   replacing positions 916-944 of the reference Cas12 protein with the amino acid sequence represented by SEQ ID NO: 11;
   wherein the amino acid sequence of the reference Cas12 protein is represented by SEQ ID NO: 1.
11. An engineered Cas12 protein or a functional derivative thereof, the sequence of which is an amino acid sequence represented by any one of SEQ ID NO: 5, 7, 9 or 12; or an amino acid sequence having 90% or more identity with the amino acid sequence represented by any one of SEQ ID NO: 5, 7, 9 or 12.
12. A detection system for detecting a target nucleic acid molecule, comprising:
   the engineered Cas12 protein or a functional derivative thereof according to any one of items 1-11;
   a guide RNA, wherein the guide RNA guides the Cas12 protein or a functional derivative thereof to specifically bind to a target nucleic acid molecule; and
   a nucleic acid probe.
13. The detection system according to item 12, wherein the nucleic acid probe is an RNA probe.
14. A method for detecting a target nucleic acid molecule in a sample, comprising:
   contacting the sample with the engineered Cas12 protein or a functional derivative thereof according to any one of items 1-11, a guide RNA, and a target nucleic acid molecule; and
   detecting the target nucleic acid molecule by measuring the detectable signal generated by cleavage of the nucleic acid probe by the engineered Cas12 protein or a functional derivative thereof.
15. The method according to item 14, wherein the nucleic acid probe is an RNA probe.
16. A detection system for selectively detecting a target nucleic acid molecule, comprising:
   two or more Cas12 proteins or the functional derivatives thereof having significant differences in cleavage specificity for RNA and DNA;
   a guide RNA, wherein the guide RNA guides the Cas12 proteins or the functional derivatives thereof to specifically bind to the target nucleic acid molecule; and
   a nucleic acid probe.
17. The detection system according to item 16, wherein at least one of the Cas12 proteins or the functional derivatives thereof is the engineered Cas12 protein or a functional derivative thereof according to any one of items 1-11.
18. The detection system according to item 17, wherein the nucleic acid probe is a DNA probe or an RNA probe.

In this application, by means of the protein mutation rational mutagenesis technology, the restriction on the recognition and cleavage of DNA substrates by RuvC in Cas12b is removed, so that DNA substrates or RNA substrates can be specifically recognized, and the problem of insufficient discrimination of DNA/RNA substrate recognition in practical applications is solved.

The engineered Cas12 protein or a functional derivative thereof according to the present application improves the reaction efficiency of Cas12 to RNA substrate after the activation of trans-cleavage activity, and improves the reporting efficiency of Cas12 using an RNA probe.

### BRIEF DESCRIPTION OF THE DRAWINGS

Fig. 1 shows the results of sequence alignment for Cas12b and Cas12g;
Fig. 2 shows the electrophoretograms of four muteins;
Fig. 3 shows the CRISPR cleavage results of DNA probes with respect to wild-type and muteins;
Fig. 4 shows the CRISPR cleavage results of DNA probes with respect to the muteins;
Fig. 5 shows the CRISPR cleavage results of RNA probes with respect to the muteins;
Fig. 6 shows the CRISPR cleavage results with respect to the muteins applied to the RPA amplification reaction system.

### DETAILED DESCRIPTION

The present application is further described below in conjunction with examples. It should be understood that, the examples are only used to further describe and illustrate the present application and are not used to limit the present application.

Unless otherwise defined, technical and scientific terms used in this specification have the same meanings as commonly understood by those skilled in the art. Although methods and materials similar or equivalent to those described herein can be used in experiments or applications, the materials and methods are described below. In case of conflict, the present specification including definitions will control, and further the materials, methods and examples are illustrative only and not limiting. The present application is further described below in conjunction with specific examples, but is not intended to limit the scope of the present application.

### Definitions

As used herein, the term "Cas12" or "Cas12 protein" includes Cas12a (also known as Cpf1), Cas12b, Cas12c, Cas12d, Cas12e, Cas12h, Cas12i, Cas12g, etc. In some embodiments, the Cas12 protein is a Cas12b protein, which is used in its broadest sense and includes a parent or reference Cas12b protein (e.g., AaCas12b having an amino acid sequence of SEQ ID NO: 1), derivatives or variants thereof, and functional fragments thereof, such as oligonucleotide binding fragments thereof.

As used herein, a "functional derivative" of a protein includes various variants or functional domains of the protein. As long as the variant or functional domain retains the function of a functional domain of the protein (whether the function is enhanced or weakened), it can be called a functional derivative of the protein. For example, for Cas12 protein, Cas12 protein variants or truncations that retain the functions of some of its domains are all functional derivatives of Cas12 protein.

As used herein, "domain" or "protein domain" refers to a portion of a protein sequence that can exist and function independently of the rest of the protein chain.

As used herein, "guide RNA", "sgRNA" and "gRNA" are used interchangeably herein, and refer to RNA that can form a complex with Cas12 protein and target nucleic acid.

As used herein, the terms "nucleic acid," "polynucleotide" and "nucleotide sequence" are used interchangeably to refer to a polymeric form of nucleotides of any length, including deoxyribonucleotides, ribonucleotides, combinations thereof, and analogs thereof. "Oligonucleotide" and "oligo-nucleotide" are used interchangeably and refer to short polynucleotides having no more than about 50 nucleotides.

As used herein, nucleic acid "complementarity" refers to the ability of one nucleic acid to form hydrogen bonds with another nucleic acid through traditional Watson-Crick base pairing. Percent complementarity refers to the percentage of residues in a nucleic acid molecule that can form hydrogen bonds (i.e., Watson-Crick base pairing) with another nucleic acid molecule (e.g., about 5, 6, 7, 8, 9, 10 out of 10 are about 50%, 60%, 70%, 80%, 90%, and 100% complementary, respectively). "Perfectly complementary" means that all consecutive residues of a nucleic acid sequence will form hydrogen bonds with the same number of consecutive residues in a second nucleic acid sequence. As used herein, "substantially complementary" refers to a degree of complementarity of at least about any of 70%, 75%, 80%, 85%, 90%, 95%, 97%, 98%, 99%, or 100% over a region of about 40, 50, 60, 70, 80, 100, 150, 200, 250 or more nucleotides, or to two nucleic acids that hybridize under stringent conditions. For a single base or a single nucleotide, according to the Watson-Crick base pairing principle, when A is paired with T or U, and C is paired with G or I, it is called complementary or matching, and vice versa; any other base pairing is called non-complementary or non-matching. Unless otherwise specified, the term "complementary" in the present application includes "completely complementary" and "substantially complementary". As long as two nucleic acid sequences can form a stable hybrid double strand through Walson-Crick base pairing, the two nucleic acid sequences are said to be "complementary", and the process of forming a stable hybrid double strand is called "complementary hybridization".

As used herein, the term "wild type" has the meaning commonly understood by those skilled in the art, referring to the typical form of an organism, strain, gene or characteristic that distinguishes it from a mutant or variant when it exists in nature. It can be isolated from resources found in nature and has not been deliberately modified.

As used herein, the terms "non-naturally occurring" or "engineered" are used interchangeably and refer to human involvement. When these terms are used to describe a nucleic acid molecule or polypeptide, they mean that the nucleic acid molecule or polypeptide is at least substantially free of at least one other component with which it is naturally associated or found in nature.

As used herein, the term "identity" is used to indicate a sequence match between two polypeptides or between two nucleic acids. When a position in two compared sequences is occupied by the same base or amino acid monomer subunit (e.g., a position in each of two DNA molecules is occupied by adenine, or a position in each of two polypeptides is occupied by lysine), then each of the molecules is identical at that position. The "percent identity" between the two sequences is a function of the number of matched positions shared by the two sequences divided by the number of positions compared x 100. For example, if 6 out of 10 positions in two sequences are matched, the two sequences are 60% identical. For example, the DNA sequences CTGACT and CAGGTT have 50% identity (3 matches out of a total of 6 positions). Typically, this comparison is performed when the two sequences are aligned to yield maximum identity. Such an alignment can be obtained, for example, by the method in Needleman et al. (1970) J. Mol. Biol. 48: 443-453, which can be conveniently implemented by a computer program such as the Align program (DNAstar, Inc.). Alternatively, the PAM 120 weighted residue table may be used, the algorithm of E. Meyers and W. Miller (Comput. Appl Biosci., 4: 11-17 (1988)) was used to integrate into the ALIGN program (version 2.0). A gap length penalty of 12 and a gap penalty of 4 are used to determine the percent identity between two amino acid sequences. In addition, the percent identity between two amino acid sequences can be determined by using the Needleman and Wunsch (J MoI Biol. 48:444-453 (1970)) algorithm integrated into the GAP program in the GCG software package (available from www.gcg.com), and using a Blossum 62 matrix or a PAM250 matrix with a gap weight of 16, 14, 12, 10, 8, 6 or 4, and a length weight of 1, 2, 3, 4, 5 or 6.

As used herein, "variant" or "mutant" is defined as a polynucleotide or polypeptide that differs from a reference polynucleotide or polypeptide, respectively, but retains the essential properties. A typical variant of a polynucleotide differs in nucleic acid sequence from a reference polynucleotide. Changes in the variant nucleic acid sequence may or may not alter the amino acid sequence of a polypeptide encoded by the reference polynucleotide. Nucleotide changes may result in amino acid substitutions, additions, deletions, fusions and truncations in the polypeptide encoded by the reference sequence, as described below. A typical variant of a polypeptide differs in amino acid sequence from a reference polypeptide. Generally, differences are limited so that the sequences of the reference polypeptide and the variant are closely similar overall and, in many regions, identical. The amino acid sequences of the variant and reference polypeptide may differ by any combination of one or more substitutions, additions, and deletions. The substituted or inserted amino acid residue may or may not be one encoded by the genetic code. Variants of a polynucleotide or polypeptide may be naturally occurring (such as allelic variants), or may be variants that are not known to occur naturally. Non-naturally occurring variants of polynucleotides and polypeptides can be prepared by mutagenesis techniques, by direct synthesis, and by other recombinant methods known to those skilled in the art.

As used herein, the term "target nucleic acid" or "target nucleic acid molecule" refers to the target nucleic acid in a sample, which may be target RNA or target DNA, or may contain both target RNA and target DNA.

The present application provides an engineered Cas12 protein or a functional derivative thereof, which comprises the following mutations:
the specificity of RuvC and/or Nuc active centers in the reference Cas12 protein for recognizing and cleaving DNA substrates is modified. Particularly, the reference Cas12 protein can be a known wild-type Cas12 protein. In some embodiments, the reference Cas12 protein can be a known wild-type Cas12 protein. In some embodiments, the reference Cas12 protein can be a wild-type Cas12b protein.

In some embodiments, the reference Cas12 protein can be a wild-type AaCas12b protein, and its amino acid sequence is represented by SEQ ID NO: 1.

In some embodiments, the specificity of the RuvC active center in the reference Cas12 protein for recognizing and cleaving DNA substrates is modified.

In some embodiments, the specificity of the Nuc active center in the reference Cas12 protein for recognizing and cleaving DNA substrates is modified.

In some embodiments, the specificity of the RuvC and Nuc active centers in the reference Cas12 protein for recognizing and cleaving DNA substrates is modified.

In some embodiments, the specificity of the RuvC and/or Nuc active centers in the reference Cas12 protein for recognizing and cleaving DNA substrates is modified as follows:
the fingers upstream and downstream of the α-helix of the gatekeeper amino acids in the reference Cas12 protein are replaced with zinc finger domains.

In some embodiments, removing the restriction on the recognition and cleavage of DNA substrates by RuvC and/or Nuc active centers in the reference Cas12 protein is replacing the domains in part or all of the region between positions 915-945, wherein the amino acid position numbering is as defined in SEQ ID NO: 1. The partial region may be one or more sites in positions 915-945, or may be one or more regions. For example, the partial region may be site P916, or site L941, or site P916 and site L941; alternatively, the partial region may be positions 919-944, or positions 916-944.

In some embodiments, removing the restriction on the recognition and cleavage of DNA substrates by RuvC and/or Nuc active centers in the reference Cas12 protein is replacing the domains in part or all of the region between positions 919-944, wherein the amino acid position numbering is as defined in SEQ ID NO: 1.

In some embodiments, removing the restriction on the recognition and cleavage of DNA substrates by RuvC and/or Nuc active centers in the reference Cas12 protein is replacing the domain in part or all of the region between positions 916-944, for example, by replacing it with a zinc finger domain, wherein the amino acid position numbering is as defined in SEQ ID NO: 1.

In some embodiments, the zinc finger domain has 1-2 zinc binding sites, and the zinc binding site is any one selected from the group consisting of: [CxxxxC], [CxxxxH], [CxxxC], [HxxxH], [CxxC] and [CxxH], wherein x represents any natural amino acid.

In some embodiments, the zinc finger domain is a zinc finger domain of Cas12g.

In some embodiments, the zinc finger domain is a zinc finger domain of HIV-1 nucleocapsid protein 7 (Ncp7).

In some embodiments, positions 919-944 of the reference Cas12 protein are replaced with the zinc finger domain of Cas12g.

In some embodiments, positions 919-944 of the reference Cas12 protein are replaced with the amino acid sequence represented by SEQ ID NO: 4, wherein the amino acid position numbering is as defined in SEQ ID NO: 1. The resulting amino acid sequence of the engineered Cas12 protein is represented by SEQ ID NO: 5.

In some embodiments, positions 919-944 of the reference Cas12 protein are replaced with the zinc finger domain of Ncp7.

In some embodiments, positions 919-944 of the reference Cas12 protein are replaced with the amino acid sequence represented by SEQ ID NO: 6, wherein the amino acid position numbering is as defined in SEQ ID NO: 1. The resulting amino acid sequence of the engineered Cas12 protein is represented by SEQ ID NO: 7.

In some embodiments, the specificity of the RuvC and/or Nuc active center in the reference Cas12 protein for recognizing and cleaving the DNA substrates is modified by replacing the gatekeeper amino acids 919-944 in the reference Cas12 protein with the amino acid sequence represented by SEQ ID NO: 8, wherein the amino acid position numbering is as defined in SEQ ID NO: 1. The resulting amino acid sequence of the engineered Cas12 protein is represented by SEQ ID NO: 9.

In some embodiments, the specificity of the RuvC and/or Nuc active center in the reference Cas12 protein for recognizing and cleaving the DNA substrates is modified by replacing the gatekeeper amino acids 916-944 in the reference Cas12 protein with the amino acid sequence represented by SEQ ID NO: 11, wherein the amino acid position numbering is as defined in SEQ ID NO: 1. The resulting amino acid sequence of the engineered Cas12 protein is represented by SEQ ID NO: 12.

The present application also provides an engineered Cas12 protein or a functional derivative thereof, which comprises any one of the following mutations based on a reference Cas12 protein:
replacing positions 919-944 of the reference Cas12 protein with the amino acid sequence represented by SEQ ID NO: 4;
replacing positions 919-944 of the reference Cas12 protein with the amino acid sequence represented by SEQ ID NO: 6;
replacing positions 919-944 of the reference Cas12 protein with the amino acid sequence represented by SEQ ID NO: 8; and
replacing positions 916-944 of the reference Cas12 protein with the amino acid sequence represented by SEQ ID NO: 11;
wherein the amino acid sequence of the reference Cas12 protein is represented by SEQ ID NO: 1.

Those skilled in the art will appreciate that, for any of the above-mentioned engineered Cas12 proteins or the functional derivatives thereof, its sequences also encompasses an amino acid sequence having 90% or more identity with the amino acid sequence represented by any one of SEQ ID NO: 5, 7, 9 or 12. Particularly, 90% or more identity can be, for example, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more.

The present application also provides a detection system for detecting a target nucleic acid molecule, the system comprises: any one or more of the above engineered Cas12 proteins or the functional derivatives thereof; a guide RNA, wherein the guide RNA guides the Cas12 protein or a functional derivative thereof to specifically bind to a target nucleic acid molecule; and a nucleic acid probe. Particularly, the target nucleic acid molecule may be target RNA or target DNA.

In some embodiments, the nucleic acid probe is an RNA probe, and the specific composition and length of the RNA probe can be designed according to actual needs.

In some embodiments, the sequence of the RNA probe is 5'FAM-rUrUrUrArGrCrArGrGrArUrUrCrArGrGrUrUrUrU-3'BHQ1 (SEQ ID NO: 17).

In some embodiments, the sequence of the RNA probe is 5'FAM-rCrArUrArUrUrGrArCrGrCrArUrArCrArArArCrArUrUrCrCrCrArCrCrArArCrArGrArGr CrCrU-3'BHQ1 (SEQ ID NO: 18).

In some embodiments, the sequence of the RNA probe is 5'FAM-rCrCrCrCrCrCrCrCrCrCrC-3'BHQ1 (SEQ ID NO: 19).

The present application also provides a method for detecting a target nucleic acid molecule in a sample, the method comprises: contacting the sample with any one or more of the above engineered Cas12 proteins or the functional derivatives thereof, a guide RNA, and a target nucleic acid molecule; and detecting the target nucleic acid molecule by measuring the detectable signal generated by cleavage of the nucleic acid probe by the engineered Cas12 protein or a functional derivative thereof. Particularly, the target nucleic acid molecule may be target RNA or target DNA.

In some embodiments, the nucleic acid probe is an RNA probe, and the specific composition and length of the RNA probe can be designed according to actual needs.

In some embodiments, the sequence of the RNA probe is 5'FAM-rUrUrUrArGrCrArGrGrArUrUrCrArGrGrUrUrUrU-3'BHQ1 (SEQ ID NO: 17).

In some embodiments, the sequence of the RNA probe is 5'FAM-rCrArUrArUrUrGrArCrGrCrArUrArCrArArArCrArUrUrCrCrCrArCrCrArArCrArGrArGr CrCrU-3'BHQ1 (SEQ ID NO: 18).

In some embodiments, the sequence of the RNA probe is 5'FAM-rCrCrCrCrCrCrCrCrCrCrC-3'BHQ1 (SEQ ID NO: 19).

The present application also provides a detection system for selectively detecting a target nucleic acid molecule, the system comprises: two or more Cas12 proteins or the functional derivatives thereof having significant differences in cleavage specificity for RNA and DNA; a guide RNA, wherein the guide RNA guides the Cas12 protein or a functional derivative thereof to specifically bind to the target nucleic acid molecule; and a nucleic acid probe. Particularly, the two or more Cas12 proteins or a functional derivative thereof can be two, three, four or more.

In some embodiments, at least one Cas12 protein or a functional derivative thereof having significant differences in cleavage specificity for RNA and DNA is any one of the engineered Cas12 proteins or a functional derivative thereof described above. Particularly, the target nucleic acid molecule may be target RNA or target DNA.

In some embodiments, the nucleic acid probe is a DNA probe or an RNA probe, and the specific composition and length of the DNA probe and the RNA probe can be designed according to actual needs.

In some embodiments, the sequence of the RNA probe is 5'FAM-rUrUrUrArGrCrArGrGrArUrUrCrArGrGrUrUrUrU-3'BHQ1 (SEQ ID NO: 17).

In some embodiments, the sequence of the RNA probe is 5'FAM-rCrArUrArUrUrGrArCrGrCrArUrArCrArArArCrArUrUrCrCrCrArCrCrArArCrArGrArGr CrCrU-3'BHQ1 (SEQ ID NO: 18).

In some embodiments, the sequence of the RNA probe is 5'FAM-rCrCrCrCrCrCrCrCrCrCrC-3'BHQ1 (SEQ ID NO: 19).

The present application discovered that, a specific domain of the Cas12 protein is a domain for specific capture of nucleic acid, which affects the ability of specific capture of nucleic acid trans-cleavage reaction substrates. Therefore, by knocking out this domain, the activity of the trans-cleavage reaction of either DNA or RNA substrates is greatly weakened. On this basis, by complementing different nucleic acid-specific binding domains, different nucleic acid capture capabilities can theoretically be formed and presented to RuvC for cleavage. In this application, the applicant attempted to replace the zinc finger structure domain of Cas12g which has a higher homology, and the zinc finger structure domain of Ncp7 which has a lower homology, in this segment. Both domains are domains that have preferential recognition for RNA -- Cas12g has been shown to have a much higher ability to cleave RNA than DNA, while Ncp7 is a structural protein that specifically binds to RNA. In this application, both of these replacements for the original Cas12b domain have been shown to have higher RNA recognition and cleavage capabilities. Furthermore, after replacing other zinc finger structures having specific base recognition ability, the resulting engineered Cas12 protein can obtain the resolution capabilities of specific recognition and cleavage of a specific base combination.

### EXAMPLES

### Example 1: Sequence Alignment and Preference Analysis for Cas12b and Cas12g

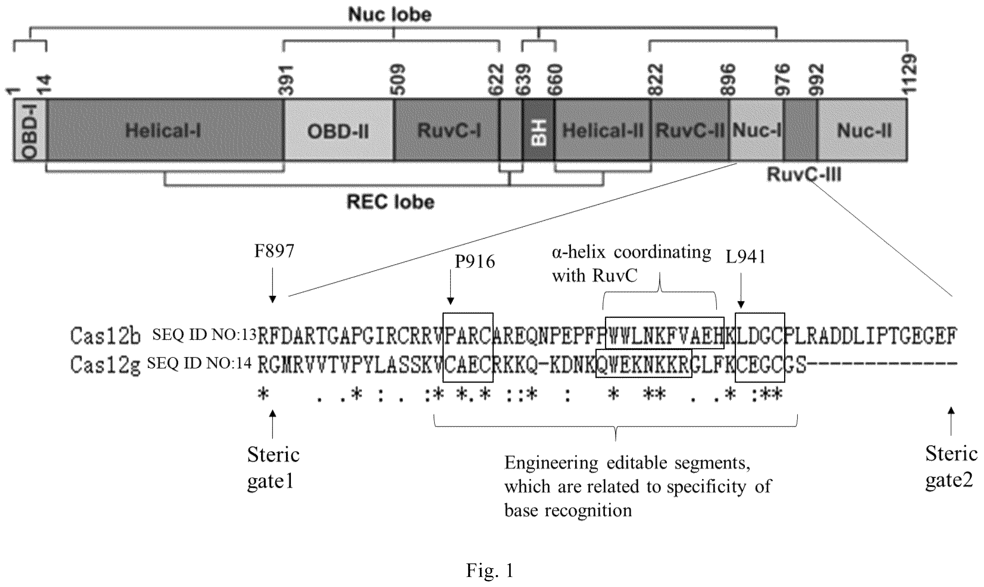

The CLUSTAL 2.1 Multi-Sequence Alignments tool was used to perform homology alignment of Cas12b and Cas12g, and the results are shown in Fig. 1.

In Fig. 1, SEQ ID NO: 13 is: 
RFDARTGAPGIRCRRVPARCAREQNPEPFPWWLNKFVAEHKLDGCPLRADDLIPTGE
GEF
 SEQ ID NO: 14 is:
RGMRVVTVPYLASSKVCAECRKKQ-KDNKQWEKNKKRGLFKCEGCGS

It was found that the two had a high degree of similarity in the RuvC (Nuv) region. The structure of the α-helix in Cas12b that cooperates with RuvC and is homologous to Cas12g1 was identified, and the potential sites P916 and L941 upstream and downstream of the α-helix that can form stronger zinc fingers were identified.

### Example 2: Finger Structure Mutations Upstream and Downstream of the α-Helix of the Gatekeeper Amino Acids of Cas12b and Acquisition of the Proteins.

The wild-type AaCas12b was mutated in the following four ways. Particularly, the amino acid sequence of wild-type AaCas12b is represented by SEQ ID NO: 1, and the amino acid sequence of wild-type Cas12g1 is represented by SEQ ID NO: 2.
SEQ ID NO: 1 
MAVKSMKVKLRLDNMPEIRAGLWKLHTEVNAGVRYYTEWLSLLRQENLYRRSPNGDGEQ
ECYKTAEECKAELLERLRARQVENGHCGPAGSDDELLQLARQLYELLVPQAIGAKGDAQQI
ARKFLSPLADKDAVGGLGIAKAGNKPRWVRMREAGEPGWEEEKAKAEARKSTDRTADVL
RALADFGLKPLMRVYTDSDMSSVQWKPLRKGQAVRTWDRDMFQQAIERMMSWESWNQR
VGEAYAKLVEQKSRFEQKNFVGQEHLVQLVNQLQQDMKEASHGLESKEQTAHYLTGRALR
GSDKVFEKWEKLDPDAPFDLYDTEIKNVQRRNTRRFGSHDLFAKLAEPKYQALWREDASF
LTRYAVYNSIVRKLNHAKMFATFTLPDATAHPIWTRFDKLGGNLHQYTFLFNEFGEGRHAIR
FQKLLTVEDGVAKEVDDVTVPISMSAQLDDLLPRDPHELVALYFQDYGAEQHLAGEFGGA
KIQYRRDQLNHLHARRGARDVYLNLSVRVQSQSEARGERRPPYAAVFRLVGDNHRAFVHF
DKLSDYLAEHPDDGKLGSEGLLSGLRVMSVDLGLRTSASISVFRVARKDELKPNSEGRVPFC
FPIEGNENLVAVHERSQLLKLPGETESKDLRAIREERQRTLRQLRTQLAYLRLLVRCGSEDVG
RRERSWAKLIEQPMDANQMTPDWREAFEDELQKLKSLYGICGDREWTEAVYESVRRVWR
HMGKQVRDWRKDVRSGERPKIRGYQKDVVGGNSIEQIEYLERQYKFLKSWSFFGKVSGQ
VIRAEKGSRFAITLREHIDHAKEDRLKKLADRIIMEALGYVYALDDERGKGKWVAKYPPCQ
LILLEELSEYQFNNDRPPSENNQLMQWSHRGVFQELLNQAQVHDLLVGTMYAAFSSRFDA
RTGAPGIRCRRVPARCAREQNPEPFPWWLNKFVAEHKLDGCPLRADDLIPTGEGEFFVSPFS
AEEGDFHQIHADLNAAQNLQRRLWSDFDISQIRLRCDWGEVDGEPVLIPRTTGKRTADSYG
NKVFYTKTGVTYYERERGKKRRKVFAQEELSEEEAELLVEADEAREKSVVLMRDPSGIINR
GDWTRQKEFWSMVNQRIEGYLVKQIRSRVRLQESACENTGDI*
SEQ ID NO:2

MAQASSTPAVSPRPRPRYREERTLVRKLLPRPGQSKQEFRENVKKLRKAFLQFNADVSGVC
QWAIQFRPRYGKPAEPTETFWKFFLEPETSLPPNDSRSPEFRRLQAFEAAAGINGAAALDDP
AFTNELRDSILAVASRPKTKEAQRLFSRLKDYQPAHRMILAKVAAEWIESRYRRAHQNWER
NYEEWKKEKQEWEQNHPELTPEIREAFNQIFQQLEVKEKRVRICPAARLLQNKDNCQYAGK
NKHSVLCNQFNEFKKNHLQGKAIKFFYKDAEKYLRCGLQSLKPNVQGPFREDWNKYLRY
MNLKEETLRGKNGGRLPHCKNLGQECEFNPHTALCKQYQQQLSSRPDLVQHDELYRKWR
REYWREPRKPVFRYPSVKRHSIAKIFGENYFQADFKNSVVGLRLDSMPAGQYLEFAFAPWP
RNYRPQPGETEISSVHLHFVGTRPRIGFRFRVPHKRSRFDCTQEELDELRSRTFPRKAQDQKF
LEAARKRLLETFPGNAEQELRLLAVDLGTDSARAAFFIGKTFQQAFPLKIVKIEKLYEQWPN
QKQAGDRRDASSKQPRPGLSRDHVGRHLQKMRAQASEIAQKRQELTGTPAPETTTDQAAK
KATLQPFDLRGLTVHTARMIRDWARLNARQIIQLAEENQVDLIVLESLRGFRPPGYENLDQE
KKRRVAFFAHGRIRRKVTEKAVERGMRVVTVPYLASSKVCAECRKKQKDNKQWEKNKKR
GLFKCEGCGSQAQVDENAARVLGRVFWGEIELPTAIP

(1) The 919-944 segment of the amino acid sequence of the wild-type AaCas12b (the amino acid sequence of this segment is CAREQNPEPFPWWLNKFVAEHKLDGC (SEQ ID NO: 3)) was replaced with the homologous segment of Cas12g1 (the amino acid sequence of this segment is CARCRKKQKDNKQWEKNKKRGLFKCEGC (SEQ ID NO: 4)). The resulting protein is called AaCas12b-SCas12g, and its amino acid sequence is represented by SEQ ID NO: 5. The underlined part indicates the replaced segment. 
SEQ ID NO:5
MAVKSMKVKLRLDNMPEIRAGLWKLHTEVNAGVRYYTEWLSLLRQENLYRRSPNGDG
EQECYKTAEECKAELLERLRARQVENGHCGPAGSDDELLQLARQLYELLVPQAIGAKGD
AQQIARKFLSPLADKDAVGGLGIAKAGNKPRWVRMREAGEPGWEEEKAKAEARKSTDR
TADVLRALADFGLKPLMRVYTDSDMSSVQWKPLRKGQAVRTWDRDMFQQAIERMMS
WESWNQRVGEAYAKLVEQKSRFEQKNFVGQEHLVQLVNQLQQDMKEASHGLESKEQT
AHYLTGRALRGSDKVFEKWEKLDPDAPFDLYDTEIKNVQRRNTRRFGSHDLFAKLAEPK
YQALWREDASFLTRYAVYNSIVRKLNHAKMFATFTLPDATAHPIWTRFDKLGGNLHQYT
FLFNEFGEGRHAIRFQKLLTVEDGVAKEVDDVTVPISMSAQLDDLLPRDPHELVALYFQD
YGAEQHLAGEFGGAKIQYRRDQLNHLHARRGARDVYLNLSVRVQSQSEARGERRPPYA
AVFRLVGDNHRAFVHFDKLSDYLAEHPDDGKLGSEGLLSGLRVMSVDLGLRTSASISVFR
VARKDELKPNSEGRVPFCFPIEGNENLVAVHERSQLLKLPGETESKDLRAIREERQRTLRQ
LRTQLAYLRLLVRCGSEDVGRRERSWAKLIEQPMDANQMTPDWREAFEDELQKLKSLY
GICGDREWTEAVYESVRRVWRHMGKQVRDWRKDVRSGERPKIRGYQKDVVGGNSIEQI
EYLERQYKFLKSWSFFGKVSGQVIRAEKGSRFAITLREHIDHAKEDRLKKLADRIIMEAL
GYVYALDDERGKGKWVAKYPPCQLILLEELSEYQFNNDRPPSENNQLMQWSHRGVFQE
LLNQAQVHDLLVGTMYAAFSSRFDARTGAPGIRCRRVPARCARCRKKQKDNKQWEKNK
KRGLFKCEGCPLRADDLIPTGEGEFFVSPFSAEEGDFHQIHADLNAAQNLQRRLWSDFDI
SQIRLRCDWGEVDGEPVLIPRTTGKRTADSYGNKVFYTKTGVTYYERERGKKRRKVFAQ
EELSEEEAELLVEADEAREKSVVLMRDPSGIINRGDWTRQKEFWSMVNQRIEGYLVKQIR
SRVRLQESACENTGDI*
(2) The 919-944 segment of the amino acid sequence of the wild-type AaCas12b was replaced with the Ncp7 zinc finger segment (the amino acid sequence of this segment is CFNCGKEGHTARNCRAPRKKGCWKCGKEGHNMKDC (SEQ ID NO: 6)). The resulting protein is called AaCas12b-NCP7, and its amino acid sequence is represented by SEQ ID NO: 7. The underlined part indicates the replaced segment. 
SEQ ID NO: 7
MAVKSMKVKLRLDNMPEIRAGLWKLHTEVNAGVRYYTEWLSLLRQENLYRRSPNGDG
EQECYKTAEECKAELLERLRARQVENGHCGPAGSDDELLQLARQLYELLVPQAIGAKGD
AQQIARKFLSPLADKDAVGGLGIAKAGNKPRWVRMREAGEPGWEEEKAKAEARKSTDR
TADVLRALADFGLKPLMRVYTDSDMSSVQWKPLRKGQAVRTWDRDMFQQAIERMMS
WESWNQRVGEAYAKLVEQKSRFEQKNFVGQEHLVQLVNQLQQDMKEASHGLESKEQT
AHYLTGRALRGSDKVFEKWEKLDPDAPFDLYDTEIKNVQRRNTRRFGSHDLFAKLAEPK
YQALWREDASFLTRYAVYNSIVRKLNHAKMFATFTLPDATAHPIWTRFDKLGGNLHQYT
FLFNEFGEGRHAIRFQKLLTVEDGVAKEVDDVTVPISMSAQLDDLLPRDPHELVALYFQD
 
YGAEQHLAGEFGGAKIQYRRDQLNHLHARRGARDVYLNLSVRVQSQSEARGERRPPYA
AVFRLVGDNHRAFVHFDKLSDYLAEHPDDGKLGSEGLLSGLRVMSVDLGLRTSASISVFR
VARKDELKPNSEGRVPFCFPIEGNENLVAVHERSQLLKLPGETESKDLRAIREERQRTLRQ
LRTQLAYLRLLVRCGSEDVGRRERSWAKLIEQPMDANQMTPDWREAFEDELQKLKSLY
GICGDREWTEAVYESVRRVWRHMGKQVRDWRKDVRSGERPKIRGYQKDVVGGNSIEQI
EYLERQYKFLKSWSFFGKVSGQVIRAEKGSRFAITLREHIDHAKEDRLKKLADRIIMEAL
GYVYALDDERGKGKWVAKYPPCQLILLEELSEYQFNNDRPPSENNQLMQWSHRGVFQE
LLNQAQVHDLLVGTMYAAFSSRFDARTGAPGIRCRRVPARCFNCGKEGHTARNCRAPRK
KGCWKCGKEGHNMKDCPLRADDLIPTGEGEFFVSPFSAEEGDFHQIHADLNAAQNLQR
RLWSDFDISQIRLRCDWGEVDGEPVLIPRTTGKRTADSYGNKVFYTKTGVTYYERERGK
KRRKVFAQEELSEEEAELLVEADEAREKSVVLMRDPSGIINRGDWTRQKEFWSMVNQRI
EGYLVKQIRSRVRLQESACENTGDI*
(3) The 919-944 segment of the amino acid sequence of the wild-type AaCas12b was replaced with the segment of the 5MPL protein (the amino acid sequence of this segment is SQRPGAHLTVKKIFVGGIKEDTEEHHLRDYFEQYGKIEVIEIMTDRGSGKKRGFAFVTFDDH DSVDKIVIQKYHTVNGHNCEVRKALSKQEMASASSSQRGR (SEQ ID NO: 8)). The resulting protein is called AaCas12b-5MPL, and its amino acid sequence is represented by SEQ ID NO: 9. The underlined part indicates the replaced segment. 
SEQ ID NO:9
MAVKSMKVKLRLDNMPEIRAGLWKLHTEVNAGVRYYTEWLSLLRQENLYRRSPNGDG
EQECYKTAEECKAELLERLRARQVENGHCGPAGSDDELLQLARQLYELLVPQAIGAKGD
AQQIARKFLSPLADKDAVGGLGIAKAGNKPRWVRMREAGEPGWEEEKAKAEARKSTDR
TADVLRALADFGLKPLMRVYTDSDMSSVQWKPLRKGQAVRTWDRDMFQQAIERMMS
WESWNQRVGEAYAKLVEQKSRFEQKNFVGQEHLVQLVNQLQQDMKEASHGLESKEQT
AHYLTGRALRGSDKVFEKWEKLDPDAPFDLYDTEIKNVQRRNTRRFGSHDLFAKLAEPK
YQALWREDASFLTRYAVYNSIVRKLNHAKMFATFTLPDATAHPIWTRFDKLGGNLHQYT
FLFNEFGEGRHAIRFQKLLTVEDGVAKEVDDVTVPISMSAQLDDLLPRDPHELVALYFQD
YGAEQHLAGEFGGAKIQYRRDQLNHLHARRGARDVYLNLSVRVQSQSEARGERRPPYA
AVFRLVGDNHRAFVHFDKLSDYLAEHPDDGKLGSEGLLSGLRVMSVDLGLRTSASISVFR
VARKDELKPNSEGRVPFCFPIEGNENLVAVHERSQLLKLPGETESKDLRAIREERQRTLRQ
LRTQLAYLRLLVRCGSEDVGRRERSWAKLIEQPMDANQMTPDWREAFEDELQKLKSLY
GICGDREWTEAVYESVRRVWRHMGKQVRDWRKDVRSGERPKIRGYQKDVVGGNSIEQI
EYLERQYKFLKSWSFFGKVSGQVIRAEKGSRFAITLREHIDHAKEDRLKKLADRIIMEAL
GYVYALDDERGKGKWVAKYPPCQLILLEELSEYQFNNDRPPSENNQLMQWSHRGVFQE
LLNQAQVHDLLVGTMYAAFSSRFDARTGAPGIRCRRVPARSQRPGAHLTVKKIFVGGIKE
DTEEHHLRDYFEQYGKIEVIEIMTDRGSGKKRGFAFVTFDDHDSVDKIVIQKYHTVNGH
NCEVRKALSKQEMASASSSQRGRPLRADDLIPTGEGEFFVSPFSAEEGDFHQIHADLNAA
QNLQRRLWSDFDISQIRLRCDWGEVDGEPVLIPRTTGKRTADSYGNKVFYTKTGVTYYE
RERGKKRRKVFAQEELSEEEAELLVEADEAREKSVVLMRDPSGIINRGDWTRQKEFWSM
VNQRIEGYLVKQIRSRVRLQESACENTGDI*
(4) The 916-944 segment of the amino acid sequence of the wild-type AaCas12b (the amino acid sequence of this segment is CAREQNPEPFPWWLNKFVAEHKLDGCPLR (SEQ ID NO: 10)) was replaced with GGGGGG (SEQ ID NO: 11). The resulting protein is called AaCas12b-ggg, and its amino acid sequence is represented by SEQ ID NO: 12. The underlined part indicates the replaced segment.

SEQ ID NO: 12
MAVKSMKVKLRLDNMPEIRAGLWKLHTEVNAGVRYYTEWLSLLRQENLYRRSPNGDG
EQECYKTAEECKAELLERLRARQVENGHCGPAGSDDELLQLARQLYELLVPQAIGAKGD
AQQIARKFLSPLADKDAVGGLGIAKAGNKPRWVRMREAGEPGWEEEKAKAEARKSTDR
TADVLRALADFGLKPLMRVYTDSDMSSVQWKPLRKGQAVRTWDRDMFQQAIERMMS
WESWNQRVGEAYAKLVEQKSRFEQKNFVGQEHLVQLVNQLQQDMKEASHGLESKEQT
AHYLTGRALRGSDKVFEKWEKLDPDAPFDLYDTEIKNVQRRNTRRFGSHDLFAKLAEPK
YQALWREDASFLTRYAVYNSIVRKLNHAKMFATFTLPDATAHPIWTRFDKLGGNLHQYT
FLFNEFGEGRHAIRFQKLLTVEDGVAKEVDDVTVPISMSAQLDDLLPRDPHELVALYFQD
YGAEQHLAGEFGGAKIQYRRDQLNHLHARRGARDVYLNLSVRVQSQSEARGERRPPYA
AVFRLVGDNHRAFVHFDKLSDYLAEHPDDGKLGSEGLLSGLRVMSVDLGLRTSASISVFR
VARKDELKPNSEGRVPFCFPIEGNENLVAVHERSQLLKLPGETESKDLRAIREERQRTLRQ
LRTQLAYLRLLVRCGSEDVGRRERSWAKLIEQPMDANQMTPDWREAFEDELQKLKSLY
GICGDREWTEAVYESVRRVWRHMGKQVRDWRKDVRSGERPKIRGYQKDVVGGNSIEQI
EYLERQYKFLKSWSFFGKVSGQVIRAEKGSRFAITLREHIDHAKEDRLKKLADRIIMEAL
GYVYALDDERGKGKWVAKYPPCQLILLEELSEYQFNNDRPPSENNQLMQWSHRGVFQE
LLNQAQVHDLLVGTMYAAFSSRFDARTGAPGIRCRRVPARGGGGGGPLRADDLIPTGEG
EFFVSPFSAEEGDFHQIHADLNAAQNLQRRLWSDFDISQIRLRCDWGEVDGEPVLIPRTT
GKRTADSYGNKVFYTKTGVTYYERERGKKRRKVFAQEELSEEEAELLVEADEAREKSVV
LMRDPSGIINRGDWTRQKEFWSMVNQRIEGYLVKQIRSRVRLQESACENTGDI*

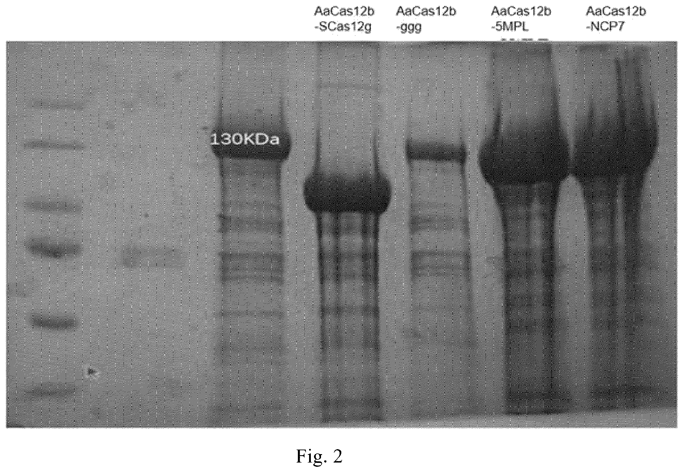

The corresponding vectors were designed according to the amino acid sequences of the above four mutants, transferred into the pET28 expression vector, and induced for expression in the *Escherichia coli* strain BL21. Four corresponding muteins were obtained through protein purification methods such as affinity chromatography, and ion exchange chromatography. Fig. 2 is a polyacrylamide gel electrophoretogram of the purified four proteins.

### Example 3: Verification of DNA Probe Cleavage Activity of Several Muteins, with Wild-type AaCas12b as a Control.

Particularly, according to the component composition shown in Table 1, each component was placed in a qPCR instrument for fluorescence value analysis, and mutant or wild-type AaCas12b, target sgRNA (150 ng, purchased from GenScript Biotech Co., Ltd., sequence: GTCTAAAGGACAGATTTTCAACGGGTGTGCCAATGGCCACTTTCCAGGTGGCAAAGCCC GTTGAACTTCAAGCGAAGTGGCACACTCAATACTTGAGCACACT (SEQ ID NO: 15)),

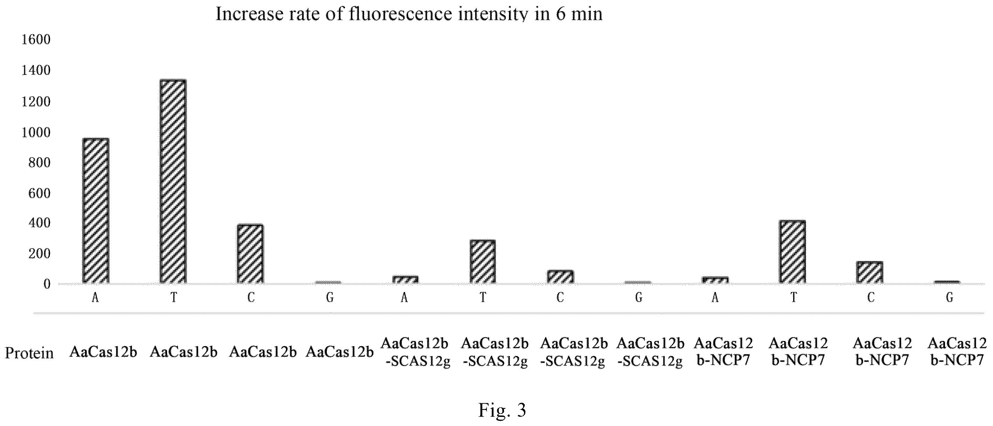

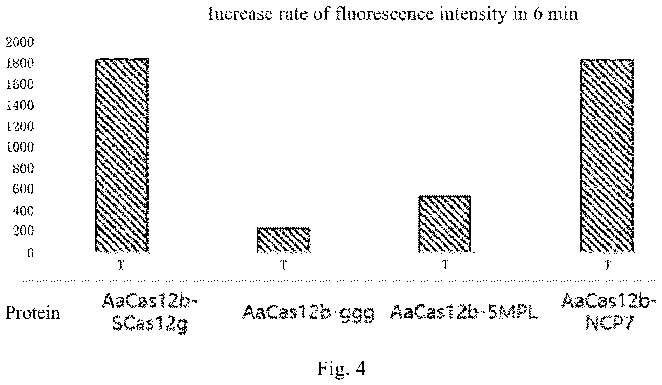

RNase inhibitor, ssDNA probe/ssRNA probe (purchased from Shanghai Biolig Biotechnology Co., Ltd.), and target synthesis template (1E12 copies, purchased from Shanghai Biolig Biotechnology Co., Ltd.) were placed in 1×rCutSmart buffer to react at 42°C for 60 min by using qPCR instrument, and the fluorescence intensity increase in the first 6 minutes was intercepted for fluorescence analysis. The detection results are shown in Figs. 3 and 4. The probe sequence information and sample sequence information are shown in Tables 2 and 3.

**Table 1: Crispr cleavage system of the novel coronavirus O target**

| Names of components | | Concentration for use | | Final concentration | | 1rxn/µL | |
|---|---|---|---|---|---|---|---|
| | ddH2O | | / | | / | to 20µL | |
| | rCutSmart | | 10X | | 1X | | 2 |
| | RNase inhibitor | | 20U/µL | | 0.5U/µL | | 0.5 |
| | Cas12b | | 600ng/µL | | 60ng /µL | | 2 |
| | sgRNA | | 300ng/µL | | 7.5ng /µL | | 0.5 |
| | probe | | 5µM | | 500nM | | 2 |
| | Sample | | 2E11copies/µL | | 1E12copies | | 5 |

**Table 2: Probe sequence information**

| | |
|---|---|
| T-FAM-BHQ1 | 5'FAM-TTTTTTT-3'BHQ1 |
| C-FAM-BHQ1 | 5'FAM-CCCCCCC-3'BHQ1 |
| A-FAM-BHQ1 | 5'FAM-AAAAAAA-3'BHQ1 |
| G-FAM-BHQ1 | 5'FAM-GGGGGGG-3'BHQ1 |

**Table 3: Sample sequence information**

| | Sequence |
|---|---|
| Sample | TAATACGACTCACTATAGGGCTTCACCTGATGCTGTTACAGCGTATAATGGT TATCTTACTTCTTCTTCTAAAACACCTGAAGAACATTTTATTGAAACCATCT CACTTGCTGGTTCCTATAAAGATTGGTCCTATTCTGGACAATCTACACAACT AGGTATAGAATTTCTTAAGAGAGGTGATAAAAGTGTATATTACACTAGTAAT CCTACCACATTCCACCTAGATGGTGAAGTTATCACCTTTGACAATCTTAAG |
| | ACACTTCTTTCTTTGAGAGAAGTGAGGACTATTAAGGTGTTTACAACAGTA GACAACATTAACCTCCACACGCAAGTTGTGGACATGTCAATGACATATGGG TGTTGCTATGCCTAATCTTTACAAAATGCAAAGAATGCTATTAGAAAAGTGT GACCTTCAAAATTATGGTGATAGTGCAACATTACCTAAAGGCATAATGATG AATGTCGCAAAATATACTCAACTGTGTCAATATTTAAACACATTAACATTAG CTGTACCCTATAATATGAGAGTTATACATTTTACACTTAACGGTCTTTGGCTT GATGACGTAGTTTACTGTCCAAGACATGTGATCTGCACCTCTGAAGACATG CTTAACCCTAATTATGAAGAAAGGTTGATACAGCCAATCCTAAGACACCTA AGTATAAGTTTGTTCGCATTCAACCAGGACAGACTTTTTCAGTGTTAGCTT GTTACAATGGTTCACCATCTGGTGTTTACCAATGTGCTATGAGGCCCAATTT CACTATTAAGGGTTCATTCCTTGTTTCTATAGATTAGCTAATGAGTGTGCTC AAGTATTGAGTGAAATGGTCATGTGTGGCGGTTCACTATATGTTAAACCAG GTGGAACCTCATCAGGAGATGCCACAACTGCTTATGCTAATAGTGTTTTTA ACATTTGTCAAGCTGTCA (SEQ ID NO: 16) |

Fig. 3 shows the CRISPR cleavage results of the DNA probe with respect to the wild-type and muteins. The results show that compared with the DNA probe cleavage activity of the wild-type Cas12b protein, the cleavage activity of the AaCas12b-SCas12g protein and the Cas12b-NCP7 protein on the DNA probe is decreased, the preference for cleavage of each base is changed, and the cleavage preference of the two muteins for the dA probe is decreased significantly.

Fig. 4 shows the CRISPR cleavage results of the DNA probe with respect to the muteins. The results show that compared with the AaCas12b-SCas12g protein and Cas12b-NCP7 protein with replaced zinc finger structure, the mutant AaCas12b-ggg with truncated replacement region and the AaCas12b-5MPL with replaced non-zinc finger structure have lower cleavage ability for DNA probe.

### Example 4: Verification of RNA Probe Cleavage Activity of Several Muteins, with Wild-type AaCas12b as a Control.

The cleavage system of Example 3 was used to test the activity of various RNA probes.

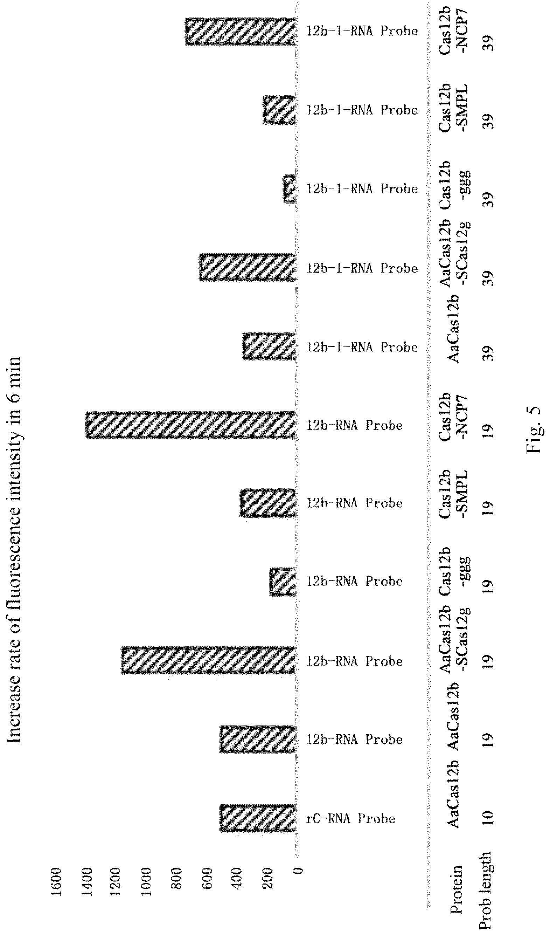

Particularly, according to the component composition of Example 3, each component was placed in a qPCR instrument for fluorescence value analysis. The mutant or wild-type AaCas12b, target sgRNA (SEQ ID NO: 15), RNase inhibitor, ssDNA probe/ssRNA probe (purchased from Shanghai Bioligo Biotechnology Co., Ltd.), and target synthesis template (1E12 copies, purchased from Shanghai Bioligo Biotechnology Co., Ltd.) were placed in 1×rCutSmart buffer to react at 42°C for 60 min by using qPCR instrument. The fluorescence intensity increase in the first 6 minutes was intercepted for fluorescence analysis, and the detection results are shown in Fig. 5. The probe sequence information is shown in Table 4.

**Table 4: Probe sequence information**

| Probes | Sequences |
|---|---|
| 12b-RNA probe | 5'FAM-rUrUrUrArGrCrArGrGrArUrUrCrArGrGrUrUrU-3'BHQ1 (SEQ ID NO: 17) |
| 12b-1-RNA probe | 5‘FAM-rCrArUrArUrUrGrArCrGrCrArUrArCrArArArArCrArUrUrCrCrCrArCrCr ArArCrArGrArGrCrCrU-3’BHQ1 (SEQ ID NO: 18) |
| rC-RNA Probe | 5'FAM-rCrCrCrCrCrCrCrCrCrC-3'BHQ1 (SEQ ID NO: 19) |

The results show that wild-type Cas12b has similar cleavage activity for RNA probes of different lengths. As for the reaction of 12b-RNA probe, the mutated AaCas12b-SCas12g and Cas12b-NCP7 proteins have higher cleavage activity than the wild-type Cas12b protein on the cleavage of various RNA probes.

### Example 5: Application of AaCas12b-SCas12g and Cas12b-NCP7 by using RNA probes for detection

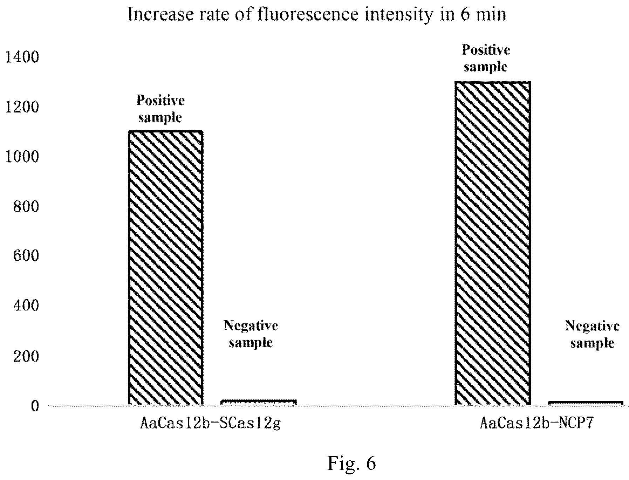

Particularly, according to the composition of the kit (purchased from Amp-Future company), the RPA system A, B buffer, RPA upstream and downstream primers (each 20 pM, purchased from Shanghai Sangon Biotech Co., Ltd.), and the actual novel coronavirus sample were placed in a qPCR instrument to incubate at 42°C for 30 minutes, wherein the real extracted novel coronavirus sample was selected as the sample. The RPA amplified components and the CRISPR cleavage system components were placed in a qPCR instrument for fluorescence value analysis according to the component composition of Example 3. The mutant or wild-type AaCas12b, target sgRNA (SEQ ID NO: 15), RNase inhibitor, and ssRNA probe (purchased from Shanghai Bioligo Biotechnology Co., Ltd.) were placed in 1×rCutSmart buffer to react at 42°C for 60 minutes by using qPCR instrument. The fluorescence intensity increase in the first 6 minutes was intercepted for fluorescence analysis, and the detection results are shown in Fig. 6. Particularly, the primer sequence information is shown in Table 5, the reaction system of RPA amplification is shown in Table 6, and the cleavage system of CRISPR reaction is shown in Table 7.

**Table 5: Sequence information of RPA primers**

| | |
|---|---|
| RPA-F | TTACTTCTTCTTCTAAAACACCTGAAGAACATT (SEQ ID NO: 20) |
| RPA-R | GTGTAGATTGTCCAGAATAGGACCAATCTTTAT (SEQ ID NO: 21) |

**Table 6: The reaction system of RPA amplification**

| Components | Volumes (µL) |
|---|---|
| A buffer | 29.4 |
| RPA-F(10µM) | 2 |
| RPA-R(10µM) | 2 |
| ddH2O and RNA template | 14.1 |
| B buffer | 2.5 |
| Total volume | 50 |

**Table 7: Cleavage system of CRISPR reaction**

| | Names of components | Concentration for use | | Final concentration | | 1 rxn/µL | |
|---|---|---|---|---|---|---|---|
| | ddH2O | | / | | / | | 13 |
| | rCutSmart | | 10X | | 1X | | 2 |
| | RNase inhibitor | | 20U/µL | | 0.5U/µL | | 0.5 |
| | Cas12b | | 600ng/µL | | 60ng/µL | | 2 |
| | sgRNA | | 300ng/µL | | 7.5ng/µL | | 0.5 |
| 12b-RNA probe (SEQ ID NO: 17) | | | 5µM | | 500nM | | 2 |
| Sample of the amplification product | | | / | | / | | 50 |

The results show that after isothermal amplification, by using specific sgRNA targeting the novel coronavirus as a guide, AaCas12b-SCas12g and Cas12b-NCP7 can correctly distinguish positive and negative novel coronavirus clinical samples with the application of 12b-RNA probe.

## Claims

1. An engineered Cas12 protein or a functional derivative thereof, comprising the following mutations:
removing the restriction on the recognition and cleavage of DNA substrates by RuvC and/or Nuc active centers in a reference Cas12 protein,
preferably, the amino acid sequence of the reference Cas12 protein is represented by SEQ ID NO: 1.

2. The engineered Cas12 protein or a functional derivative thereof according to claim 1, wherein removing the restriction on the recognition and cleavage of DNA substrates by RuvC and/or Nuc active centers in the reference Cas12 protein is achieved by:
replacing the fingers upstream and downstream of the α-helix of the gatekeeper amino acids in the reference Cas12 protein with zinc finger domains.

3. The engineered Cas12 protein or a functional derivative thereof according to claim 1 or 2, wherein removing the restriction on the recognition and cleavage of DNA substrates by RuvC and/or Nuc active centers in the reference Cas12 protein is replacing the domains in part or all of the region in positions 915-945, wherein the amino acid position numbering is as defined in SEQ ID NO: 1.

4. The engineered Cas12 protein or a functional derivative thereof according to claim 2, wherein the zinc finger domain has 1-2 zinc binding sites, and the zinc binding site is any one selected from the group consisting of: [CxxxxC], [CxxxxH], [CxxxC], [HxxxH], [CxxC] and [CxxH], wherein x represents any natural amino acid.

5. The engineered Cas12 protein or a functional derivative thereof according to claim 4, wherein the zinc finger domain is a zinc finger domain of Cas12g or HIV-1 nucleocapsid protein 7 (Ncp7).

6. The engineered Cas12 protein or a functional derivative thereof according to claim 5, wherein positions 919-944 of the reference Cas12 protein are replaced with the amino acid sequence represented by SEQ ID NO: 4, wherein the amino acid position numbering is as defined in SEQ ID NO: 1.

7. The engineered Cas12 protein or a functional derivative thereof according to claim 5, wherein positions 919-944 of the reference Cas12 protein are replaced with the amino acid sequence represented by SEQ ID NO: 6, wherein the amino acid position numbering is as defined in SEQ ID NO: 1.

8. The engineered Cas12 protein or a functional derivative thereof according to claim 1, wherein positions 919-944 of the reference Cas12 protein are replaced with the amino acid sequence represented by SEQ ID NO: 8, wherein the amino acid position numbering is as defined in SEQ ID NO: 1.

9. The engineered Cas12 protein or a functional derivative thereof according to claim 1, wherein positions 916-944 of the reference Cas12 protein are replaced with the amino acid sequence represented by SEQ ID NO: 11, wherein the amino acid position numbering is as defined in SEQ ID NO: 1.

10. An engineered Cas12 protein or a functional derivative thereof, comprising any one of the following mutations based on a reference Cas12 protein:
replacing positions 919-944 of the reference Cas12 protein with the amino acid sequence represented by SEQ ID NO: 4;
replacing positions 919-944 of the reference Cas12 protein with the amino acid sequence represented by SEQ ID NO: 6;
replacing positions 919-944 of the reference Cas12 protein with the amino acid sequence represented by SEQ ID NO: 8; and
replacing positions 916-944 of the reference Cas12 protein with the amino acid sequence represented by SEQ ID NO: 11;
wherein the amino acid sequence of the reference Cas12 protein is represented by SEQ ID NO: 1.

11. An engineered Cas12 protein or a functional derivative thereof, the sequence of which is an amino acid sequence represented by any one of SEQ ID NO: 5, 7, 9 or 12; or an amino acid sequence having 90% or more identity with the amino acid sequence represented by any one of SEQ ID NO: 5, 7, 9 or 12.

12. A detection system for detecting a target nucleic acid molecule, comprising:
the engineered Cas12 protein or a functional derivative thereof according to any one of claims 1-11;
a guide RNA, wherein the guide RNA guides the Cas12 protein or a functional derivative thereof to specifically bind to a target nucleic acid molecule; and
a nucleic acid probe.

13. The detection system according to claim 12, wherein the nucleic acid probe is an RNA probe.

14. A method for detecting a target nucleic acid molecule in a sample, comprising:
contacting the sample with the engineered Cas12 protein or a functional derivative thereof according to any one of claims 1-11, a guide RNA, and a target nucleic acid molecule; and
detecting the target nucleic acid molecule by measuring the detectable signal generated by cleavage of the nucleic acid probe by the engineered Cas12 protein or a functional derivative thereof.

15. The method according to claim 14, wherein the nucleic acid probe is an RNA probe.

16. A detection system for selectively detecting a target nucleic acid molecule, comprising:
two or more Cas12 proteins or a functional derivative thereof having significant differences in cleavage specificity for RNA and DNA;
a guide RNA, wherein the guide RNA guides the Cas12 protein or a functional derivative thereof to specifically bind to the target nucleic acid molecule; and
a nucleic acid probe.

17. The detection system according to claim 16, wherein at least one of the Cas12 proteins or the functional derivatives thereof is the engineered Cas12 protein or a functional derivative thereof according to any one of claims 1-11.

18. The detection system according to claim 17, wherein the nucleic acid probe is a DNA probe or an RNA probe.
